# EUROPEAN PATENT SPECIFICATION

(11) **EP 1 080 734 B1**
(45) Date of publication and mention of the grant of the patent: **01.12.2004**
(21) Application number: 00126830.9
(22) Date of filing: 04.05.1989
(51) Int. Cl.: A61L 2/20, A61L 2/10, A61L 2/08

(54) **Sterilization**
Sterilisierung
Stérilisation

(30) Priority: 05.05.1988 GB 8810603
(43) Date of publication of application: 07.03.2001
(62) Divisional of application: 95203637.4
(73) Proprietor: ELOPAK SYSTEMS AG, 8152 Glattbrugg (CH)
(72) Inventor: Castberg, Helge Bakketun, 1410 Kolbotn (NO); Chant, Nigel Anthony, Stotfold, Bedfordshire (GB)
(74) Representative: Burrows, Anthony Gregory

(56) References cited:
- CH-A- 387 881
- US-A- 4 309 388
- US-A- 4 366 125
- US-A- 4 792 407
- DATABASE WPI Section Ch, Week 8538 Derwent Publications Ltd., London, GB; Class J04, AN 85-234441 XP002013352 & JP 60 153982 A (TOSHIBA KK), 13 August 1985 (1985-08-13)
- PATENT ABSTRACTS OF JAPAN vol. 012, no. 022 (C-470), 22 January 1988 (1988-01-22) & JP 62 176595 A (EBARA INFILCO CO LTD;OTHERS: 01), 3 August 1987 (1987-08-03)

## Description

This invention relates to a method of sterilization. It has general application, but particular application to packaging.

Derwent File Supplier No. AN 85-234441(JP60153982) appears to disclose the removal of contamination compounds from the surfaces of articles by flowing water containing dissolved ozone over the surfaces under the irradiation of UV. The water can contain dissolved ozone or bubbles of ozonized gas.

Where the contamination compounds are organic compounds, they are partially oxidized by the treatment, aldehydes, ketones and carboxylic acids being formed, and removed by the water. Where the contamination compounds are inorganic compounds, they dissolve or disperse in the water and are thus removed.

Ozone is decomposed under the irradiation of UV to form an oxygen radical, an hydroxyl radical and a hydrogen peroxide radical. As such radicals have high activity butshort life-time, the treatment generates the radicals on the surfaces themselves and so makes them most effective. The washing effect is further promoted because the reactivity of the contamination compounds can be enhanced by the UV irradiation, thus breaking down the compounds into smaller particles and into more hydrophilic compositions. The washing effect was enhanced with increasing washing time and with increasing ozone dissolved concentration. Use of oxygen gas in ozone generation increased the ozone concentration and enhanced the washing effect.

Such treatment is concerned with removing contamination from the surface of articles and is not concerned with sterilization in which micro-organisms are rendered non-viable.

US4309388 discloses sterilizing apparatus which comprises an enclosure surrounding the top, bottom and sides of a central sterilizing enclosure space and adjacent inlet and outlet enclosure spaces opening horizontally to the exterior of the enclosure at the opposite ends thereof. A continuously moving conveyor carries open-topped glass bottles to be sterilized horizontally through the inlet, sterilizing and outlet enclosure spaces of the enclosure. The sterilizing enclosure space is divided by partition walls into an upper compartment which opens at the bottom thereof into a lower compartment immediately above the path of travel of the open tops of the bottles passing through the lower compartment of the sterilizing enclosure space. Ozone-generating ultraviolet lamps are mounted in the upper compartment and a blower circulates air between the lower and upper compartments and through the opening of the upper compartment into the lower compartment so that ozone generated in the upper compartment is directed into the openings at the tops of the bottles moving in the lower compartment. Ozone decomposition accelerating ultra-violet lamps are positioned in the inlet and outlet enclosure spaces to accelerate decomposition of the ozone in the air escaping to the surrounding atmosphere through the inlet and outlet enclosure spaces.

The UV employed for the purpose of indirectly sterilizing the bottles has a radiation wavelength which is predominantly that in the wavelength band which generates ozone and not in the wavelength band which kills micro organisms. Although UV radiation predominantly in the wavelength band which kills micro-organisms is employed, it is not employed for the purpose of rendering the micro-organisms non-viable but simply in order to degrade the ozone after its production.

CH387881 discloses as known processes the use of gases or vapours with bactericidal properties, ozone for example or certain formolic products. It discloses as its invention a sterilization system in which the objects to be sterilized are subjected in an enclosure to the simultaneous actions of (1) vapours of a bactericidal substance, for example Methanogene, Trioxymethylene and Aldylene, (2) ozone generated in the enclosure by a lamp radiating at a wavelength of 185 nm. and (3) UV radiation of a wavelength of 253.7 nm.

In CH387881, the primary action of the ozone is particularly for the benefit of the operator of the system, in that it deodorizes the vapours from the bactericidal substance and removes their causticity from them, without however modifying their toxicity. This primary action is accompanied by a secondary action in which the effect of the ultraviolet radiation is to produce an activation of the bactericidal action, not only of the ozone but also of the bactericidal vapours.

Patent Abstracts of Japan, Vol.12, No.22 (C-470) (2869) (JP 62-176595A) discloses a method of decomposing a low molecular organic substance by adding ozonated water to a mixture of hydrogen peroxide, and waste water containing the organic substance, while the whole is irradiated with UV. It is not directed to the sterilization of micro-organisms.

In US4366125, a long sheet material to be sterilized is passed through an atmosphere of H₂O₂ mist of low concentration and of droplet particle size of approximately 10 microns at room temperature for approximately one second and is then irradiated for approximately one second with UV lamps positioned to irradiate opposite surfaces of the sheet material, whereby the material is sterilized as a result of the synergistic effect produced by the combination of these two sterilization steps.

According to the present invention, there is provided a method of sterilization as defined in claim 1.

We have found that the use of the UV in the ozone atmosphere instead of in air gives an improved killing efficiency of micro-organisms. Moreover, we have not needed to employ a bactericidal substance in addition to the ozone.

In the present specification, "ozone" means a gas comprised of significant amounts of O₃ radicals and hydroxyl radicals.

The ozone employed is preferably produced by subjecting air to a photoflux in the vacuum ultraviolet range, which photons have between 8-10 ev and ionize only the oxygen content, forming various activated oxygen compounds in the gas phase. Such activated oxygen is advantageously PHOTOZONE (Registered Trade Mark) gas which is produced by the PHOTOZONE (Registered Trade Mark) lamp obtainable from Water Management A/S of Oslo, Norway.

Such activated oxygen has a relatively greater oxidation potential than ozone produced by the classical method of employing a high voltage of between 8,000 and 10,000 volts across electrodes, and thus a greater killing power of micro-organisms, owing to its higher proportion of hydroxyl radicals.

The present invention is particularly useful for the sterilization of food contact surfaces. Such surfaces can be the inside surfaces of walls, sheets, films, cups and cartons. Where they are the inside surfaces of cartons ready to be filled, they may be for example liquid contact surfaces of cartons formed from paperboard (or from paperboard and aluminium foil, or from paperboard and polymer) with internal and external plastics coatings. Such liquid can be long-life milk or orange juice, for example.

Tests in which the food contact surfaces of cartons were subjected to UV-C and were contained in an ozone atmosphere appear to show a synergistic effect between the UV-C and the ozone.

An important commercial advantage of the invention is that the required kill rates of exposed micro-organisms are achieved by treatment times significantly less than with known sterilization methods.

In order that the invention may be clearly understood and readily carried into effect, examples of known sterilization treatments and of sterilization treatments according to the invention will now be described.

### EXAMPLES 1 - 7

These Examples set forth in Table I are a selection of various comparable tests and illustrate the killing efficiency of exposure of B. subtilis var. globigii (B-17) to UV-C (254 nm), 2% H₂O₂, and/or heat, under various atmospheres. Air is mentioned at the commencement of all Examples because it was the initial atmosphere and remained so unless and until purged by the introduction of steam, or ozone, which latter remains the atmosphere unless and until purged by steam.

The tests were performed in gable-top, aluminium-foil-lined cartons, not top-sealed. The distance from the UV-C light source and the H₂O₂ spray nozzle tip to the base of the cartons was a maximum of 28 cm.

Cartons were prior to the test inoculated with the spore suspension to a level of approximately 10₆ spores per carton. Rinsing with plating was used to determine the spore load of the carton.

After exposure to the various combinations of treatment, cartons were treated with catalase to remove residual H₂O₂. Cartons were then covered with nutrient agar on the inside and incubated for counting survivors.

The conditions and exposure times for the tests are given in Table 1 together with the results, the various treatments in each Example being given in their actual sequence.

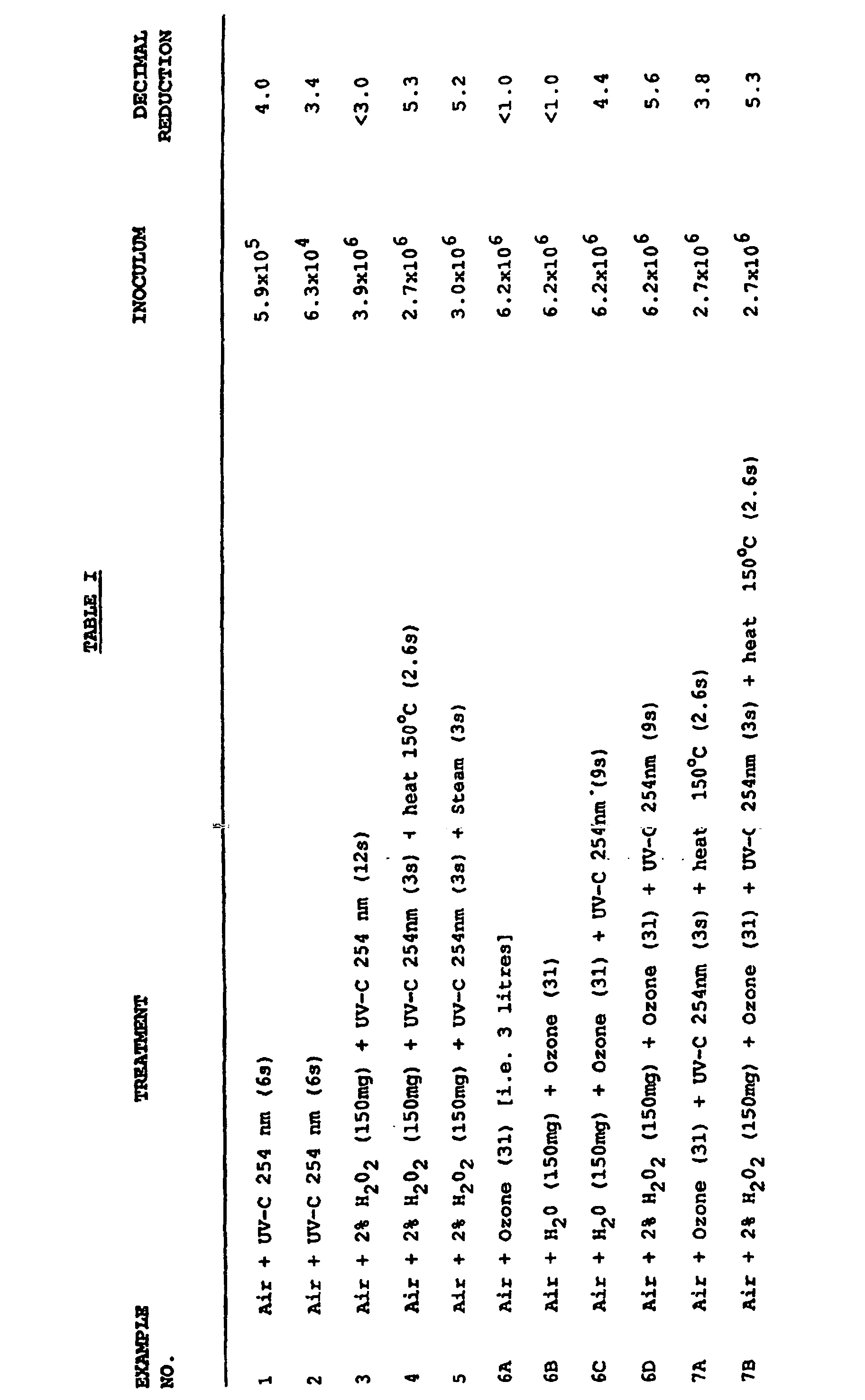

In each appropriate Example, the carton was flushed with Photozone, supplied at 0.8 l/s, for 5.0s.

Examples 6A to 6D illustrate the very greatimprovement obtained by the passage of UV-C through a moist ozone atmosphere and the further improvement obtained by the employment of an H₂O₂ spray instead of a water spray. Examples 7A and 7B illustrate the great improvement obtained by prior addition of H₂O₂ compared with passing UV-C through a dry ozone atmosphere.

Examples 1 to 5 are illustrative of known treatments. Example 6C is illustrative of the present invention. The time periods given in each Example are sequential rather than concurrent.

Testing was also carried out on the spores Aspergillus niger as these are known to be highly resistant to ultra-violet radiation. These tests were carried out in the form of a control to help justify previous results.

### Spore Preparation

Aspergillus niger spores were inoculated on Malt extract agar and incubated at 25oC for one week.

Spores were harvested using a sterile loop and suspended in 1/4 strength Ringer's solution with Tween 80 to achieve a concentration of approximately 10⁵-10⁶ spores/ml. Spores were applied to the surfaces by means of a cotton wool swab. Carton surfaces were dried overnight followed by exposure to the sterilizing test conditions.

Bacillus subtilis var.globigii B17 spores were grown and isolated.

### UV-C - Source

The UV-C source was IWASAKI irradiation equipment (254nm - UV-C) consisting of a high-intensity sterilizer GHS 490145, nine lamp sets GHL 400-2 and three irradiator sets GE 4301.

Ozone was produced by a Photozone lamp (185 nm - UV-C) from Water Management A/S.

### Methods of Inoculation and Organisms

B.subtilis var. globigii (B17) are known to be highly resistant to hydrogen peroxide and ultra-violet radiation.

The method of producing a carton loading of 10⁶ was as follows. A stock suspension of approximately 109/ml dilution was sprayed into the carton in 0.5 ml doses, where the residual in the carton is approximately 10⁶. The cartons were then dried overnight, and the verification of actual bacteriological loading was carried out on 3/4 cartons. 9 ml of 0.25 strength Ringer's solution was poured into the carton, the latter closed and shaken vigorously and approximately 20 seconds were allowed for the solution to collect into the base of the carton. 1 ml. was removed by pipette and further dilutions were carried out.

### Spray System

It was decided to use a device already in use, which proved to give excellent coverage on a 1 litre "Pure-Pak" carton. This device is commercially available from Metal Box plc of Reading, Berkshire, United Kingdom.

| Spray Parameters | |
|---|---|
| Inlet air pressure | = 90 psi. (6.4 kg/sq cm.) |
| Atomising Air | = 80 psi. (5.6 kg/sq cm.) |
| Pulse air pressure | = 60 psi. (4.2 kg/sq cm.) |
| Tank pressure | = 20 psi. (1.5 kg/sq cm.) |
| Discharge air pressure | = 10 psi. (1.05 kg/sq cm.) |
| Nozzle tip to carton base | = 270 - 280 mm. |

This setting remained standard throughout all of the testing where spraying of H₂O₂ was required. The only change was to the duration of the spraying time, which was varied to give differing levels of H₂O₂ within the carton.

## Claims

1. A method of sterilization which comprises subjecting micro-organisms simultaneously to both ozone and to UV radiation predominantly in the wavelength band which kills micro-organisms, in order to render the micro-organisms non-viable, wherein the subjecting of the micro-organisms simultaneously to the ozone and to the UV radiation obtains a synergistic effect between the UV radiation and the ozone insofar as the rendering of the micro-organisms non-viable is concerned and a further bactericidal substance is not employed.

2. A method according to claim 1, in which said wavelength band is between 220 and 330 nanometres.

3. A method according to claim 1 or 2, wherein the micro-organisms are subjected simultaneously to the ozone and to the UV radiation as aforesaid and also to moisture.

4. A method according to claim 3, wherein said moisture is provided by a spray of water or by steam.

5. A method according to any preceding claim, wherein said micro-organisms are subjected simultaneously to the ozone and to the UV radiation as aforesaid while at a surface of a packaging material.

## Patentansprüche

1. Verfahren zum Sterilisieren, bei dem Mikroorganismen gleichzeitig sowohl Ozon als auch UV-Strahlung hauptsächlich in dem Wellenlängenbereich, der Mikroorganismen abtötet, ausgesetzt werden, um die Mikroorganismen lebensunfähig zu machen, wobei die Tatsache, dass die Mikroorganismen gleichzeitig sowohl Ozon als auch UV-Strahlung ausgesetzt sind, hinsichtlich des Lebensunfähigmachens der Mikroorganismen zu einem Synergieeffekt zwischen der UV-Strahlung und dem Ozon führt und keine weitere bakterizide Substanz verwendet wird.

2. Verfahren nach Anspruch 1, bei dem der Wellenlängenbereich zwischen 220 und 330 Nanometer liegt.

3. Verfahren nach Anspruch 1 oder 2, bei dem die Mikroorganismen gleichzeitig Ozon und UV-Strahlung, wie oben genannt, und ferner Feuchtigkeit ausgesetzt werden.

4. Verfahren nach Anspruch 3, bei dem die Feuchtigkeit durch Versprühen von Wasser oder Dampf bereitgestellt wird.

5. Verfahren nach einem der vorhergehenden Ansprüche, bei dem die Mikroorganismen gleichzeitig Ozon und UV-Strahlung, wie oben genannt, ausgesetzt werden, während sie sich auf der Oberfläche eines Verpackungsmaterials befinden.

## Revendications

1. Un procédé de stérilisation qui comprend le fait de soumettre des microorganismes simultanément à la fois à de l'ozone et un rayonnement UV prédominamment dans la bande des longueurs d'onde qui tue les microorganismes, de manière à rendre les microorganismes non viables, dans lequel le fait de soumettre les microorganismes simultanément à l'ozone et au rayonnement UV permet d'obtenir un effet de synergie entre le rayonnement UV et l'ozone pour autant qu'est concerné le fait de rendre non viable les microorganismes, une autre substance bactéricide n'étant pas employée.

2. Un procédé selon la revendication 1, dans lequel ladite bande de longueurs d'onde est entre 220 et 330 nanomètres.

3. Un procédé selon la revendication 1 ou 2, dans lequel les microorganismes sont soumis simultanément à l'ozone et au rayonnement UV comme indiqué précédemment et également à de l'humidité.

4. Un procédé selon la revendication 3, dans lequel ladite humidité est assurée par une pulvérisation d'eau ou par de la vapeur d'eau.

5. Un procédé selon une quelconque revendication précédente, dans lequel lesdits microorganismes sont soumis simultanément à l'ozone et au rayonnement UV comme indiqué précédemment alors qu'ils se trouvent à la surface d'une matière d'emballage.
